(19) Europäisches Patentamt / European Patent Office / Office européen des brevets

(11) **EP 2 072 521 A1**

(12) # EUROPEAN PATENT APPLICATION

(43) Date of publication:
**24.06.2009 Bulletin 2009/26**

(51) Int Cl.:
*C07F 15/00* [(2006.01)]    *A61K 31/28* [(2006.01)]
*A61P 35/00* [(2006.01)]

(21) Application number: **07150276.9**

(22) Date of filing: **20.12.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicants:
• **Freie Universität Berlin**
  **14195 Berlin (DE)**
• **Ruhr-Universität Bochum**
  **44801 Bochum (DE)**

(72) Inventors:
• **GUST, Ronald**
  **14513, Teltow (DE)**

• **OTT, Ingo**
  **5301, Eugendorf (AT)**
• **SHELDRICK, William S.**
  **40489, Düsseldorf (DE)**
• **HARLOS, Melanie**
  **44879, Bochum (DE)**

(74) Representative: **Ziebig, Marlene et al**
  **Gulde Hengelhaupt Ziebig & Schneider**
  **Patentanwälte Rechtsanwälte**
  **Wallstrasse 58/59**
  **10179 Berlin (DE)**

(54) **Octahedral metal (III) polypyridyl complexes and their use in prevention and treatment of cancer**

(57) The present invention relates to new octahedral metal (III) polypyridyl complexes of general formula I

$$Me(hal)_3(sol)(pp) \qquad (I)$$

where
Me
represents rhodium or iridium
hal
is a halogenide selected from the group consisting of chlorine, bromine, fluorine and iodine or a pseudohalogenide selected from the group consisting of SCN, NCO or $N_3$,
sol
is a solvent selected from the group consisting of DMSO, $H_2O$, $CH_3OH$, DMF and $CH_3CN$
and
pp
is a polypyridyl ligand selected from the group constisting of 2,2'-bipyridine (bpy), 1,10-phenanthroline (phen), dipyrido[3,2-f:2',3'-h]quinoxaline (dpq), dipyrido[3,2-a:2',3'-c]phenazine (dppz) and benzo[i]dipyrido[3,2-a:2',3'-c]phenazine (dppn), optionally substituted with one or more of the substituents selected from the group constisting of hydroxy, -COOR, -$SO_3H$, -CHO, -$CH_3$, -$CF_3$, -$OCH_3$, -$OC_2H_5$, -$NO_2$, -CN, -$NH_2$, phenyl and halogenide, wherein R = H, -$CH_3$ or -$C_2H_5$, and their use in prevention and treatment of cancer.

**EP 2 072 521 A1**

**Description**

The present invention relates to new octahedral trihalido metal (III) polypyridyl complexes and their use as anticancer and antimetastatic agents.

The trichloridorhodium (III) complexes $mer,cis$-[RhCl$_3$(DMSO-κS)(Im)$_2$] (DMSO = (CH$_3$)$_2$SO, Im = imidazole) and $mer,cis$-[RhCl$_3$(DMSO-κS)$_2$(L)] (L = Im, NH$_3$) have been studied by Mestroni et al. in 1998 [1] who established cytotoxic activity (IC$_{50}$ = 1.5 ± 0.4, 0.4 ± 0.2, 9 μM) for the latter amine complex towards the human cell lines A 2780 (ovarian carcinoma), LoVo (colon carcinoma) and Calu (lung carcinoma). Replacing ammonia by imidazole leads to an increase in the IC$_{50}$ values by about an order of magnitude and $mer,cis$-[RhCl$_3$(DMSO-κS)(Im)$_2$] is essentially inactive against A2780 and Calu (IC$_{50}$ > 200 μM) and only moderately active towards the colon carcinoma cell line LoVo (IC$_{50}$ = 40 ± 15 μM). Activity against mouse P 388 leukemia has, however, been reported for the analogous compound $mer,cis$-[RhCl$_3$(DMSO-κS)(py)$_2$] (py = pyridine)[2]. The 2,2':6',2"-terpyridine (tpy) complexes $mer$-[RhCl$_3$(tpy)] and [Rh(Im)(tpy)$_2$]Cl·3H$_2$O also exhibit cytotoxicity [3], as does the compound fac-[RhCl$_3$(9-[ane]-NS$_2$)] (9-[ane]-NS$_2$ = 1-aza-4,7-dithiacyclononane)[4].

Cytotoxic activity towards the human cell lines MCF-7 (breast cancer) and HT-29 (colon cancer) (IC$_{50}$ = 2.3 (0.4) and 7.4(0.9) μM)has also been found for the iridium (III) complex [(η$^5$-C$_5$Me$_5$)IrCl(dppz)](CF$_3$SO$_3$) (dppz = dipyrido [3,2-$a$:2',3'-c]phenazine) (C$_5$Me$_5$ = pentamethylcyclopentadienyl) [5, 6]. However, in general, iridium (III) complexes are even more inert than their Rh(III) analogues and [ImH][$trans$-IrCl$_4$(Im)$_2$], [ImH][$trans$-IrCl$_4$(DMSO)(Im)] (Im = imidazole) and [(DMSO)$_2$H] [$trans$-IrCl$_4$(DMSO)$_2$] have all been found to be biologically inactive [7, 8]. These findings are in accordance with the general tenet, that the lack of reactivity of many Ir(III) complexes will correlate with an effective absence of cytotoxic effects on tumour cell lines, even at relatively high concentrations.

It is an object of the present invention to provide alternative compounds having potential to prevent and treat cancer and its metastases, preferably breast and colorectal cancer.

The problem of the invention is solved by the provision of the embodiments as defined in the claims of the present invention. It has been found that the new octahedral metal (III) polypyridyl complexes of general formula I

$$\text{Me(Hal)}_3\text{(sol)(pp)} \qquad \text{(I)}$$

where

Me    represents rhodium or iridium

hal    is a halogenide selected from the group consisting of chlorine, bromine, fluorine and iodine or a pseudohalogenide selected from the group consisting of SCN, NCO or N$_3$,

sol    is a solvent selected from the group consisting of DMSO, H$_2$O, CH$_3$OH, DMF and CH$_3$CN and

pp    is a polypyridyl ligand selected from the group constisting of 2,2'-bipyridine (bpy), 1,10-phenanthroline (phen), dipyrido[3,2-f:2',3'-h]quinoxaline (dpq), dipyrido[3,2-a:2',3'-c]phenazine (dppz) and benzo[i]dipyrido[3,2-a:2',3'-c] phenazine (dppn), optionally substituted with one or more of the substituents selected from the group constisting of hydroxy, -COOR, -SO$_3$H, -CHO, -CH$_3$, -CF$_3$, -OCH$_3$, -OC$_2$H$_5$, -NO$_2$, -CN, -NH$_2$, phenyl and halogenide, wherein R = H, -CH$_3$ or -C$_2$H$_5$,

and their physiologically tolerated isomers,
and hydrates, solvates and salts thereof
are useful cytotoxic agents for prevention and treatment of cancer and its metastases. Object of the invention are the complexes and their isomers and the hydrates, solvates and salts of the complexes and the isomers. The compounds of the invention exhibit significant cytotoxic effects which are superior to those of known cytostatic metal containing drugs as for instance cisplatin.

According to the invention, sol in formula I preferably means DMSO or H$_2$O.

Hal according to formula I means preferably chlorine or bromine and pseudohalogenide means preferably SCN.

According to the invention the preferred transition metal of the complex is rhodium.

With regard to the polypyridyl ligands of the complexes of the invention phen, dpq, dppz or dppn are preferred and dpq, dppz or dppn are especially preferred. Rhodium complexes of formula I with dpq, dppz or dppn als polypyridyl ligand exhibit superior cytotoxic effects in cell cultures which are orders of magnitude stronger than for cisplatin (compare Tab. 1). The same applies to iridium complexes of formula I with dppz and dppn as polypyridyl ligand (compare Tab. 2).

Optionally the polypyridyl ligands may be substituted with one or more of the substituents selected from the group consisting of one or more of the substituents selected from the group consisting of hydroxy, -COOR, -SO$_3$H, -CHO, -CH$_3$, -CF$_3$, -OCH$_3$, -OC$_2$H$_5$, -NO$_2$, -CN, -NH$_2$, phenyl and halogenide, wherein R = H, -CH$_3$ or -C$_2$H$_5$. The substituents -COOR, -OH, -CHO and - SO$_3$H are especially preferred to improve the solubility of the compounds of the invention in aqueous solutions, if necessary.

Particularly, the polypyridyl ligand bpy may be monosubstituted with $CH_3$, $OCH_3$, OEt, Ph, CHO, CN, COOH, $NH_2$, $NO_2$, OH, $SO_3H$, Cl or Br in position 3, 4 or 5 or disubstituted in positions 3,3'; 4,4'; 5,5'; 4,6 or 3,5 with these substituents.

The polypyridyl ligand phen may be monosubstituted in positions 2, 3, 4 or 5 or disubstituted in positions 2,9; 3,8; 4,7 or 5,6 with $CH_3$, $OCH_3$, OEt, Ph, CHO, CN, COOH, $NO_2$, OH, $SO_3H$, Cl or Br.

The polypyridyl ligand dpq may bear one of substituents -COOR, -CHO, -$CH_3$, halogenide, hydroxy, phenyl, -CN or -$NH_2$ in position 2 or 11 or two of these substituents in positions 2,9; 4,7; 11,12; 3,8. Preferred dpq complexes are such with -$CH_3$, -CHO or -$CH_2OH$ in 2-position and in positions 11 and 12 - $COOC_2H_5$. Especially preferred dpq compounds are such with -COOH disubstituted in 4, 4'-position or in 11, 12-position or disubstituted with -CN in 11, 12-position.

The polypyridyl ligand dppz may be disubstuted in 12, 13-position with -$CH_3$, - CN, -$NO_2$, halogenide or phenyl, in 2, 9-position with -$NH_2$, -$CH_3$, -COOH or chloride, in 11, 14-position with phenyl and bromide, in 3, 8-position with chloride, in 3, 4-position with -$CH_3$ and in 4, 7-position with $CH_3$ or phenyl. According to the invention dppz complexes with -$CF_3$ in 2-position and hydroxy or halogenide in 4-position are also suitable. Monosubstituted dppz complexes with halogenide in 2-position, hydroxy or methyl in position 11 or -COOH, -$NH_2$,

- $NO_2$, halogenide or $OCH_3$ in 12-position can also be used according to the invention.
 Particularly, the polypyridyl ligand dppn may be substituted with alkyl (for instance -$CH_3$ or n-butyl) in 2, 9-position.
 In an embodiment of the invention the complexes are hydrates or solvates, preferably of formula Ia

$$[Me(hal)_3(sol)(pp)] \cdot (CH_3OH)_m \cdot (H_2O)_n, \qquad (Ia)$$

wherein m is 0; 1 or 2 and n is 0; 1; 1,5; 2 or 3.

In an other embodiment of the invention the complexes are isomers, especially fac- and mer-isomers, and hydrates or solvates of the isomers, preferably of formula Ia. Preferred fac and mer isomers of the invention are mer-$RhCl_3(DMSO)(pp)$, fac-$RhCl_3(DMSO)(pp)$, mer-$RhCl_3(DMSO-H_2O)(pp)$, fac-$RhCl_3(DMSO-H_2O)(pp)$, fac-$RhCl_3(H_2O)(pp)$, mer-$RhCl_3(H_2O)(pp)$, fac-$IrCl_3(DMSO)(pp)$, mer-$IrCl_3(DMSO)(pp)$, fac-$IrBr_3(H_2O)(pp)$, mer-$IrBr_3(H_2O)(pp)$, fac-$IrCl_3(H_2O)(pp)$, mer-$IrCl_3(H_2O)(pp)$.

Especially preferred isomeric complexes of the present invention are

mer-$RhCl_3(DMSO)(bpy)$,
mer-$RhCl_3(OMSO)(phen) \cdot H_2O$, mer-$RhCl_3(DMSO)(dpq)$,
mer-$RhCl_3(DMSO)(dppz) \cdot 1,5H_2O$, mer-$RhCl_3(DMSO)(dppn)$,
fac-$[IrCl_3(OMSO)(bpy)] \cdot 2H_2O$, fac-$[IrCl_3(OMSO)(phen) \cdot CH_3OH \cdot H_2O$,
fac-$[IrCl_3(OMSO)(dpq)] \cdot 3H_2O$, fac-$[IrCl_3(OMSO)(dppz)] \cdot 2H_2O$,
fac-$[IrCl_3(OMSO)(dppn) \cdot 2CH_3OH$, fac-$[IrBr_3(H_2O)(phen)]$.

The above mentioned compounds of the invention also include the pharmaceutically acceptable salts, or any other compound which, upon administration to the human subject, is capable of providing (directly or indirectly) the therapeutically active agent.

Salts according to the invention which may be conveniently used in therapy include physiologically acceptable base salts, eg derived from an appropriate base, such as alkali metal (e.g. sodium) salts, alkaline earth metal (e.g. magnesium) salts or ammonium salts.

The complexes of formula I are new compounds which general preparation procedure is described in the Examples of the present invention. Complexes not mentioned in the examples can be prepared in analogy using the corresponding starting compounds.

The complexes mer-$RhCl_3(DMSO)(pp)$ may be prepared by reaction of mer,cis-$RhCl_3(DMSO-\kappa S)_2(DMSO-\kappa O)$[12-14] with the appropriate polypyridyl ligand (bpy, phen, dpq, dppz, dppn) in $CH_3OH/H_2O$ solution at about 75°C. Crystals of the fac-$RhCl_3(DMSO)(pp)$ isomers may be grown over a period of about 7 days by slow evaporation of water/methanol solutions of the corresponding mer-isomers.

In complexes fac-$IrCl_3(DMSO)(pp)$ may be prepared by stepwise reaction of $IrCl_3 \cdot 3H_2O$ with equimolar quantities of the appropriate polypyridyl ligand and DMSO in methanol solution in the dark. Crystals of the corresponding mer-$IrCl_3(DMSO)(pp)$ isomers may be obtained by slow evaporation of water/methanol solutions of the corresponding fac-isomers.

According to another embodiment the present invention provides pharmaceutical compositions comprising in an amount sufficient to exhibit a therapeutic effect as active substance one or more octahedral metal (III) polypyridyl complexes of formula I.

The pharmaceutical compositions may also comprise conventional auxiliary substances, preferably carriers, adjuvants and/or vehicles. For example, said carriers can be fillers, extenders, binders, humectants, disintegrants, dissolution retarders, absorption enhancers, wetting agents, adsorbents, and/or lubricants.

The pharmaceutical compositions of the invention may be prepared as a gel, powder, tablet, sustained-release tablet, premix, emulsion, infusion formulation, drops, concentrate, granulate, syrup, pellet, bolus or capsule and/or used in this form.

For example, the pharmaceutical composition of the present invention can be administered orally in any orally tolerable dosage form, including capsules, tablets and aqueous suspensions and solutions, without being restricted thereto. In case of tablets for oral application, carriers frequently used include microcrystalline cellulose, lactose and corn starch. Typically, lubricants such as magnesium stearate can be added. For oral administration in the form of capsules, useful diluents such as lactose and dried corn starch are employed. In oral administration of aqueous suspensions the active substance is combined with emulsifiers and suspending agents. Also, particular sweeteners and/or flavors and/or coloring agents can be added, if desired.

The complexes of formula I can also be present in micro-encapsulated form, optionally with one or more of the above-specified carriers.

In addition to the complexes of formula I as active substance(s), ointments, pastes, creams and gels may include conventional carriers such as animal and vegetable fats, waxes, paraffins, starch, tragacanth, cellulose derivatives, polyethylene glycols, silicones, bentonites, silicic acid, talc and zinc oxide or mixtures of these substances.

If the pharmaceutical composition according to the invention is provided as solution or emulsion it may include conventional carriers such as solvents, solubilizers, and emulsifiers such as water, ethyl alcohol, isopropyl alcohol, ethyl carbonate, ethyl acetate, benzyl alcohol, benzyl benzoate, propylene glycol, 1,3-butylene glycol, dimethylformamide, oils, especially cotton seed oil, peanut oil, corn oil, olive oil, castor oil and sesame oil, glycerol, glycerol formal, tetrahydrofurfuryl alcohol, polyethylene glycols, and fatty esters of sorbitan, or mixtures of these substances. For parenteral application, the solutions and emulsions may also be present in a sterile and blood-isotonic form.

In addition to the complexes of formula I as active substance(s), suspensions may include conventional carriers such as liquid diluents, e.g. water, ethyl alcohol, propylene glycol, suspending agents, e.g. ethoxylated isostearyl alcohols, polyoxyethylene-sorbitol and sorbitan esters, microcrystalline cellulose, aluminum metahydroxide, bentonite, agar, and tragacanth, or mixtures of these substances.

The pharmaceutical compositions can be present in the form of a lyophilized sterile injectable formulation, e.g. as a sterile injectable aqueous solution or aqueous or oily suspension. Such a suspension can also be formulated by means of methods known in the art, using suitable dispersing or wetting agents (such as Tween 80) and suspending agents.

The production of the pharmaceutical formulations specified above proceeds in a usual manner according to well-known methods, e.g. by mixing the active substance(s) with the carrier(s).

For administration, the metal complexes of formula I may be encapsulated in semi-solid nanoparticles which prototypes are liposomes which serve as delivery systems. Liposomes are completely closed lipid bilayer membranes enclosing an aqueous volume. Liposomes can be unilamellar vesicles (i.e. having a single membrane bilayer) or multilamellar vesicles (i.e. onion-like structures characterized by several membrane bilayers each of which is separated from the next one by an aqueous layer). The production of liposomes from saturated and unsaturated lipids has been described in a large number of patents, as well as their use as delivery systems for drugs. The metal complexes may be encapsulated therein in a per se known manner. Such liposomes are mostly made from phospholipids. Alternatively, the metal complexes according to the invention may also be encapsulated in alginates or other gel-like structures.

According to the invention the complexes of formula I are incorporated in a pharmaceutical formulation at a concentration of 0.1 to 99.5, preferably 0.5 to 95, and more preferably 20 to 80 wt.-%. That is, the active substance is present in the above pharmaceutical formulations, e.g. tablets, pills, granulates and others, at a concentration of preferably 0.1 to 99.5 wt.-% of the overall mixture.

According to a further embodiment of the invention the complexes of formula I are useful for the manufacture of a pharmaceutical composition for prevention and treatment of cancer, especially of breast or colorectal cancer and their metastases. In a preferred embodiment of the invention these complexes are useful for preventing and treating breast cancer.

A further proliferative disease which can be treated or prevented by the complexes of formula I is a neoplasia, such as for instance leukaemia. Leukaemias include, but are not limited to, acute myelogenous leukaemia, chronic myelogenous leukaemia and juvenile myelomonocytic leukaemia.

According to a further embodiment the present invention provides a method for treating cancer comprising adminstering to a patient suffering from cancer a therapeutically effective and safe amount of metal complexes of general formula I.

"Therapeutically effective amount" means an amount effective to yield the desired therapeutic response. For example, an amount effective to delay the growth of a cancer, to shrink or not metastasize. "Safe amount" refers to the quantity of a component that does not cause undue adverse side effects (such as toxicity, irritation, or allergic response) commensurate with a reasonable benefit/risk ratio when used in the manner of this invention.

Without intending to be limiting, the invention will be explained in more detail with reference to the following examples.

**Materials and Instrumentation**

UV/Vis spectra were recorded with an Analytik Jena SPECORD 200 spectrometer and CD spectra with a Jasco J-715 instrument in the range 220 - 500 nm for 1:10 complex/[DNA] mixtures [complex = 20 $\mu$M, DNA concentration in M(nucleotide) = 200 $\mu$M] in a 10 mM phosphate buffer at pH 7.2. 1 % DMSO was added to assure solubility of **Rh1a - Rh5a.** LSIMS spectra (liquid secondary ion mass spectrometry) were registered for the mass range $m/z$ < 3000 with a Fisons VG autospec employing a caesium ion gun (voltage 17 kV) and 3-nitrobenzyl alcohol as the liquid matrix. A Bruker DRX 400 was employed for the registration of $^1$H and $^{13}$C NMR spectra with chemical shifts reported as $\delta$ values relative to the signal of tetramethylsilane. Atomic absorption spectrometric measurements were performed on a Vario 6 (Analytik Jena) and elemental analyses on a Vario EL (Elementar Analysensysteme). $RhCl_3 \cdot 3H_2O$ and $IrCl_3 \cdot H_2O$ were purchased from Chempur, 1,10-phenanthroline (phen) from Acros, and 2,2'-bipyridine (bpy) and dimethylsulfoxide (DMSO) from J. T. Baker. The polypyridyl ligands dpq [9], dppz [10] und dppn [11] were prepared in accordance with literature procedures as was the starting compound $mer,cis$-[RhCl$_3$(DMSO-$\kappa S$)$_2$(DMSO-$\kappa O$)] [12] and the complexes [($\eta^5$-C$_5$Me$_5$)IrCl(dppz)](CF$_3$SO$_3$) [5] and [($\eta^5$-C$_5$Me$_5$)Ir{(Me$_2$N)$_2$CS}(dppn)](CF$_3$SO$_3$) [5].

**X-ray Structural Analyses**

Intensity data for $fac$-[RhCl$_3$(DMSO-$\kappa S$)(bpy)]·H$_2$O **Rh1b** were collected using $\omega$ scans on a Siemens P4 diffractometer equipped with graphite-monochromated MoK$\alpha$ radiation ($\lambda$ = 0.71073 Å, 4° $\leq$ 2$\theta$ $\leq$ 50°). The data were corrected semi-empirically for absorption ($\Psi$ scans) and the structures were solved by direct methods and refined by full-matrix least squares against $F_0^2$ using SHELX97 (G.M. Sheldrick, SHELXS97 and SHELXL97, Göttingen, Germany, 1997). Anisotropic temperature factors were employed for the non-hydrogen atoms with the exception of the disordered water oxygen atom of **Rh1b** and protons were included at geometrically calculated positions as riding atoms. The final R factors were R$_1$ = 0.047 and 0.033 for I > 2$\sigma$ (I) with wR$_2$ = 0.119 and 0.083 for all independent reflections.

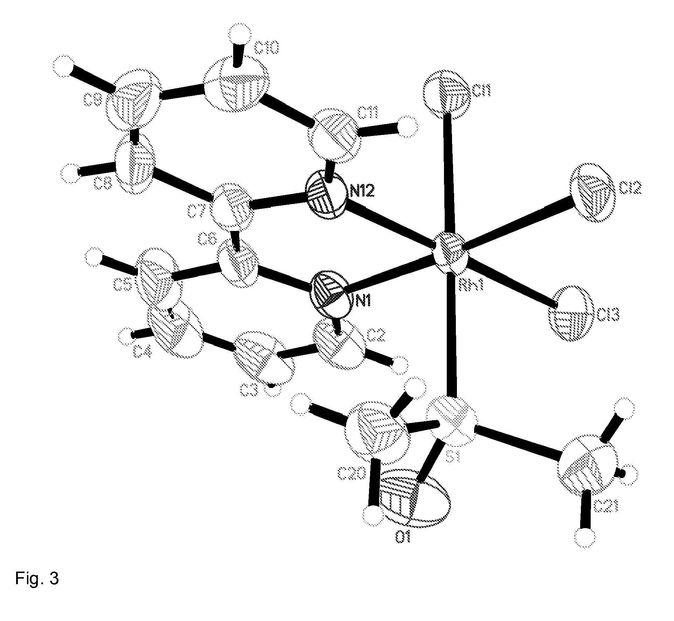

The molecular structure of **Rh1b** as established by X-ray structural analysis is depicted in Fig. 3.

**Example 1: Synthesis of rhodium (III) polypyridyl complexes of type mer-and fac-RhCl$_3$(DMSO)(pp)**

The rhodium(III) complexes $mer$-RhCl$_3$(DMSO-$\kappa S$)(pp) **Rh1a-Rh5a** (pp = bpy, phen, dpq, dppz, dppn) were prepared by treatment of the precursor $mer,cis$-[RhCl$_3$(DMSO-$\kappa S$)$_2$(DMSO-$\kappa O$)] [12-14] with an equivalent of the appropriate polypyridyl ligand in CH$_3$OH/H$_2$O solution (1/1). The general procedure is described below for **Rh1a.**

$mer$-[RhCl$_3$(DMSO)(bpy)] Rh1a. $mer,cis$-[RhCl$_3$(DMSO-$\kappa O$)(DMSO-$\kappa S$)$_2$] (200 mg, 0.45 mmol) was dissolved in 10 mL of a 1:1 mixture of methanol and water. After addition of 2,2'-bipyridine (70.3 mg, 0.45 mmol), the reaction mixture was stirred for 2 h at 75°C and then left to stand at 4°C for a further 24 h. The resulting yellow precipitate was filtered off, treated with 5 mL methanol and reprecipitated by addition of diethyl ether. The solid was filtered off, washed and dried in vacuo. Yield: 76 %. Anal. (C$_{12}$H$_{14}$Cl$_3$N$_2$ORhS) C, H, N: calcd. 32.49, 3.18, 6.32; found 32.30, 3.27, 6.36. LSIMS: $m/z$ (%) 407(100) [M-Cl]$^+$, 372(37) [M-2Cl]$^+$. $^1$H NMR (CDCl$_3$): $\delta$ 3.71 (s, 6H, CH$_3$), 7.63 (dd, 1H), 7.71 (dd, 1H), 8.04 (dd, 1H), 8.11 (d, 1H) 8.14 (dd, 1H), 8.16 (d, 1H), 10.01 (d, 2H). Crystals of $fac$-[RhCl$_3$(DMSO-$\kappa S$)(bpy)]·H$_2$O **Rh1b** suitable for X-ray analysis (and characterized by X-ray analysis) were grown over a period of 7 days by slow evaporation of a solution of **Rh1a** in water/methanol.

$mer$-[RhCl$_3$(DMSO)(phen)]·H$_2$O Rh2a. Synthesis as for **Rh1** with 1,10-phenanthroline (81.1 mg, 0.45 mmol). Yield: 74 %. Anal. (C$_{14}$H$_{16}$Cl$_3$N$_2$O$_2$RhS) C, H, N: calcd. 34.62, 3.32, 5.75; found 34.41, 3.10, 5.99. LSIMS: $m/z$ (%) 431 (100) [M-Cl]$^+$, 391(38) [M-2Cl]$^+$. $^1$H NMR (CDCl$_3$): $\delta$ 3.78 (s, 6H, CH$_3$) 7.93 (dd, 1H), 8.03 (dd, 1H), 8.04 (dd, 1H), 8.05 (d, 1H) 8.50 (dd, 1H), 8.58 (d, 1H), 10.17 (d, 2H), 10.24 (d,1H).

$mer$-[RhCl$_3$(DMSO)(dpq)] Rh3a. Synthesis as for **Rh1** with dipyrido[3,2-f:2',3'-h]quinoxaline (104.7 mg, 0.45 mmol). Yield: 76 %. Anal. (C$_{16}$H$_{14}$Cl$_3$N$_4$ORhS) C, H, N: calcd. 36.98, 2.72, 10.78; found 37.05, 2.96, 10.79. LSIMS: $m/z$ (%) 483(28) [M-Cl]$^+$, 405(9) [M-Cl-DMSO]$^+$, 370(100) [M-2Cl-DMSO]$^+$, 335(83) [M-3Cl-DMSO]$^+$. $^1$H NMR (CDCl$_3$): $\delta$ 3.84 (s, 6H, CH$_3$), 8.13 (dd, 1H), 8.23 (dd, 1H), 9.19 (s, 2H), 9.70 (d, 1H) 9.78 (d, 1H), 10.33 (d, 1H), 10.39 (d, 1H).

$mer$-[RhCl$_3$(DMSO)(dppz)]·1.5H$_2$O Rh4a. Synthesis as for **Rh1** with dipyrido[3,2-a:2',3'-c]phenazine (127.3 mg, 0.45 mmol). Yield: 71 %. Anal. (C$_{20}$H$_{19}$Cl$_3$N$_4$O$_{2.5}$RhS) C, H, N: calcd. 40.26, 9.37, 3.21 found 40.56, 8.97, 3.31.

LSIMS: $m/z$ (%) 533(19) [M-Cl]$^+$, 458(9) [M-Cl-DMSO]$^+$, 420(65) [M-2Cl-DMSO]$^+$, 385(100). $^1$H NMR (CDCl$_3$): δ 3.75 (s, 6H, CH$_3$) 8.00 (dd, 1H), 8.02 (dd, 1H), 8.11 (s, 1H), 8.13 (s, 1H) 8.38 (d, 1H), 8.40 (d, 1H), 9.76 (d, 1H), 9.82 (d, 1H), 10.22 (d, 1H), 10.28 (d, 1H) ppm.

*mer*-[RhCl$_3$(DMSO)(dppn)] **Rh5a.** Synthesis as for **Rh1** with benzo[i]dipyrido[3,2-*a*:2',3'-c]phenazine (149.6 mg, 0.45 mmol). Yield: 68 %. Anal. (C$_{24}$H$_{18}$Cl$_3$N$_4$ORhS) C, H, N: calcd. 46.51, 2.93, 9.04; found 46.42, 3.08, 8.96. LSIMS: $m/z$ (%) 583(38) [M-Cl]$^+$, 548(11) [M-2Cl]$^+$ 435(41) [M-3Cl-DMSO]$^+$. $^1$H NMR (CDCl$_3$): δ 3.80 (s, 6H, CH$_3$) 7.68, 7.70 (2d, 2H), 8.07 (dd, 1 H), 8.16 (dd, 1 H), 8.25, 8.27 (2d, 2H) 9.06, 9.08 (2s, 2H) 9.80, 9.86 (2d, 2H), 10.25, 10.31 (2d, 2H).

The *mer*-isomers of **Rh1a - Rh5a** are stable in chloroform solution, those of **Rh1a** and **Rh2a** isomerize rapidly to a mixture of fac- and mer-isomers in DMSO.

Both isomerization and rapid DMSO/H$_2$O exchange are observed for aqueous solutions of the complexes. Hence, complexes of the type mer-RhCl$_3$(DMSO-H$_2$O)(pp) and/or fac-RhCl$_3$(H$_2$O)(pp) will probably be the biologically active species.

UV/Vis and CD studies of the interaction of **Rh1a - Rh5a** with calf thymus DNA are in accordance with an absence of intercalation and time dependent $^1$H NMR indicates that the complexes do not react with the guanine N7 atom of 5'-GMP$^{2-}$.

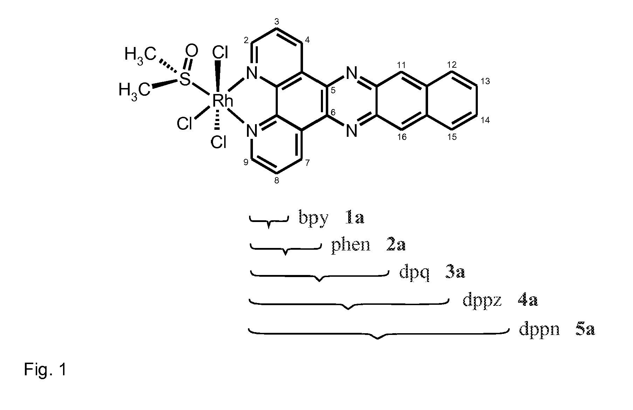

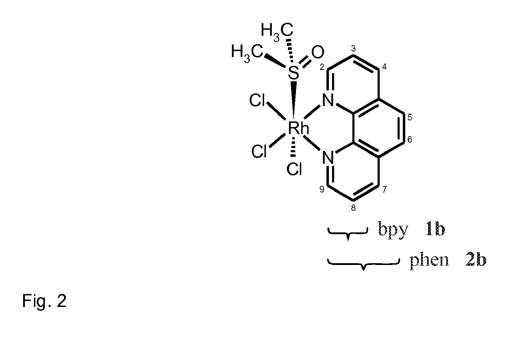

The structures of the mer complexes **Rh1a - Rh5a** and of the fac complexes **Rh1b - Rh2b** are shown in Figures 1 and 2.

**Example 2: Cytotoxicity Measurements of mer-RhCl$_3$(DMSO)(pp) complexes**

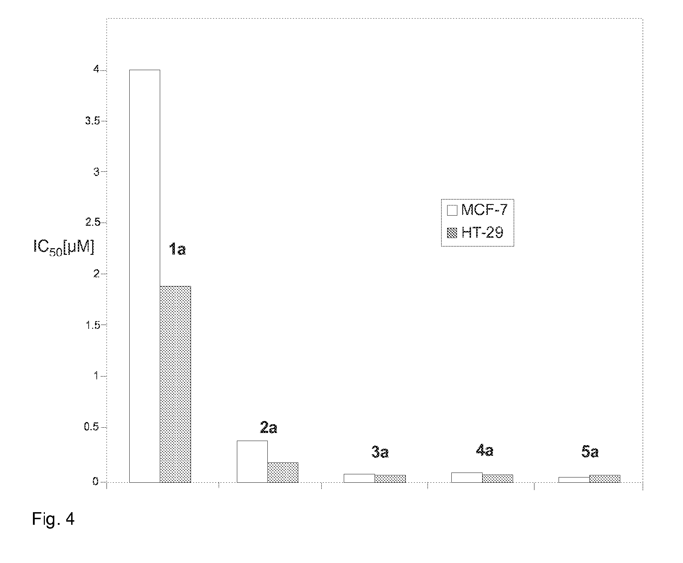

MCF-7 breast cancer and HT-29 human colon carcinoma cells were maintained in 10 % (v/v) fetal calf serum containing cell culture medium (minimum essential eagle supplemented with 2.2 g NaHCO$_3$, 110 mg/L sodium pyruvate and 50 mg/L gentamicin sulfate adjusted to pH 7.4) at 37°C/5 % CO$_2$ and passaged twice a week according to standard procedures. The antiproliferative effects of **Rh1a - Rh5a** were determined by an established procedure [15]. Cells were suspended in cell culture medium (MCF-7: 10000 cells/mL, HT-29: 2850 cells/mL), and 100 μL aliquots thereof were plated in 96 well plates and incubated at 37°C/5 % CO$_2$ for 72 h (MCF-7) or 48 h (HT-29). Stock solutions of the compounds in DMSO were freshly prepared and diluted with cell culture medium to the desired concentrations (final DMSO concentration: 0.1 % v/v). The medium in the plates was replaced with the medium containing the compounds in graded concentrations (six replicates). After further incubation for 96 h (MCF-7) or 72 h (HT-29) the cell biomass was determined by crystal violet staining and the IC$_{50}$ values were established as those concentrations causing 50 % inhibition of cell proliferation. Results were calculated from 2 - 3 independent experiments. The results are summarized in Tab. 1 and Fig. 4.

**Table 1** IC$_{50}$ values (μM) for the complexes *mer*-[RhCl$_3$(DMSO)(pp)] **Rh1a - Rh5a**

| Complex | pp | MCF-7 IC$_{50}$ | HT-29 IC$_{50}$ |
|---------|-----|-----------------|------------------|
| **Rh1a** | bpy | 4.0(0.5) | 1.9(0.5) |
| **Rh2a** | phen | 0.40(0.06) | 0.19(0.05) |
| **Rh3a** | dpq | 0.079(0.012) | 0.069(0.021) |
| **Rh4a** | dppz | 0.095(0.020) | 0.073(0.017) |
| **Rh5a** | dppn | 0.051(0.012) | 0.070(0.008) |
| **Cisplatin** | | 2.0(0.3) | 7.0(2.0) |
| **dppz** | | 0.8(0.6) | 1.8(0.2) |

It is apparent for the complexes **Rh1a - Rh3a** that their IC$_{50}$ values (bpy > phen > dpq) are strongly correlated to the surface area of the polypyridyl ligand. Whereas a dramatic improvement is observed for the smaller bpy, phen and dpq ligands of **Rh1a - Rh3a,** no further significant increase in cytotoxicity is apparent for the larger dppz and dppn ligands of **Rh4a** and **Rh5a.**

Complexes **Rh3a - Rh5a** are extremely potent cytotoxic agents with IC$_{50}$ values in the range 0.069 - 0.079 μM, that are some two orders of magnitude lower than for cisplatin.

**Example 3: Synthesis of iridium (III) polypyridyl complexes of the type fac- and mer-IrCl$_3$(DMSO)(pp)**

The iridium (III) complexes fac-IrCl$_3$(DMSO)(pp) **Ir1a - Ir5a** were prepared by stepwise reaction of IrCl$_3$.3H$_2$O with equimolar quantities of the appropriate polypyridyl ligand (pp=bpy, phen, dpq, dppz, dppn) and DMSO in CH$_3$OH

solution in the dark. The general procedure is described below for **Ir1a.**

### *fac-[IrCl$_3$(DMSO)(bpy)·2H$_2$O (Ir1a)*

31.2 mg (0.2 mmol) of 2,2'-bipyridine were added to a solution of 70.5 mg (0.2 mmol) of IrCl$_3$·3H$_2$O in 10 mL methanol. After stirring at 80°C for 2 h in the dark, 0.2 mmol (14.3 μL) DMSO were added and the mixture was heated for a further 2 h at 80°C. Following cooling to 25°C and solvent removal under vacuum, the resulting solid was washed with methanol and diethyl ether, and dried in the dark in vacuum. Yield: 70.5 mg (62 %). Anal. Calcd. for C$_{12}$H$_{14}$Cl$_3$IrN$_2$OS·2H$_2$O (M = 568.9): C 25.33, H 3.19, N 4.92: Found C 25.2, H 3.2, N 4.4 %. LSIMS: *m/z* (%) = 532(18) [M]$^+$, 497(100) [M-HCl]$^+$, 460(27) [M-2HCl]$^+$, 419(23) [M-HCl-DMSO]$^+$. $^1$H NMR (400 MHz, CD$_2$Cl$_2$, 30°C in the dark): δ = 3.44 (s, 6H, DMSO-CH$_3$), 7.71 (dd, 2H, H3/H8), 8.23 (dd, 2H, H4/H7), 8.44 (d, 2H, H5/H6), 9.14 (d, 2H, H2/H9) ppm. $^1$H NMR (400 MHz, D$_2$O, 30°C): δ = 3.50 (s, 6H, DMSO-CH$_3$), 7.56 (dd, 2H, H3/H8), 8.04 (dd, 2H, H4/H7), 8.08 (d, 2H, H5/H6), 8.66 (d, 2H, H2/H9) ppm.

### *fac-[IrCl$_3$(DMSO)(phen)]·CH$_3$OH·H$_2$O (Ir2a)*

Preparation as for **Ir1a** with 36.0 mg (0.2 mmol) of 1,10-phenanthroline. Yield: 76.5 mg (63 %). Anal. Calcd. for C$_{14}$H$_{14}$Cl$_3$IrN$_2$OS·CH$_3$OH·H$_2$O (M = 607.0): C 29.68, H 3.32, N 4.62: Found C 29.7, H 3.3, N 4.5 %. LSIMS: *m/z* (%) = 556(9) [M]$^+$, 521(30) [M-HCl]$^+$, 443(15) [M-HCl-DMSO]$^+$. $^1$H NMR (400 MHz, CD$_2$Cl$_2$, 30°C in the dark): δ = 3.50 (s, 6H, DMSO-CH$_3$), 8.01 (dd, 2H, H3/H8), 8.07 (s, 2H, H5/H6), 8.66 (d, 2H, H4/H7), 9.64 (d, 2H, H2/H9) ppm. $^1$H NMR (400 MHz, D$_2$O, 30°C): δ = 3.51 (s, 6H, DMSO-CH$_3$), 7.81 (dd, 2H, H3/H8), 7.95 (s, 2H, H5/H6), 8.48 (d, 2H, H4/H7), 9.05 (d, 2H, H2/H9) ppm. Crystals of *mer*-[IrCl$_3$(DMSO)(phen)] Ir2b were grown by slow evaporation of an H$_2$O/CH$_3$OH solution. $^1$H NMR (400 MHz, CD$_2$Cl$_2$, 30°C, in the dark): δ = 3.68(s, 6H, DMSO-CH$_3$), 7.89, 8.05 (2dd, 2H, H3/H8), 8.07 (2d, 2H, H5/H6), 8.44, 8.57 (2d, 2H, H4/H7), 9.98 (d, 1H, H2), 10.29 (d, 1H, H9) ppm.

### *fac-[IrCl$_3$(DMSO)(dpq)·3H$_2$O (Ir3a)*

Preparation as for **Ir1a** with 46.4 mg (0.2 mmol) of dipyrido[3,2-*f*:2',3'-h]quinoxaline. Yield: 87.5 mg (66 %). Anal. Calcd. for C$_{16}$H$_{14}$Cl$_3$IrN$_4$OS·3H$_2$O (M = 663.0): C 28.99, H 3.04, N 8.45: Found C 28.8, H 3.1, N 8.5 %. LSIMS: *m/z* (%) = 610(9) [M]$^+$, 573(35) [M-HCl]$^+$, 495(29) [M-HCl-DMSO]$^+$. $^1$H NMR (400 MHz, CD$_2$Cl$_2$, 30°C in the dark): δ = 3.49 (s, 6H, DMSO-CH$_3$), 7.85 (dd, 2H, H3/H8), 9.01 (s, 2H, H11/H12), 9.33 (d, 2H, H4/H7), 9.55 (d, 2H, H2/H9) ppm. $^1$H NMR (400 MHz, CD$_3$OD, 30°C): δ = 3.49 (s, 6H, DMSO-CH$_3$), 8.14 (dd, 2H, H3/H8), 9.17 (s, 2H, H11/H12), 9.28 (d, 2H, H4/H7), 9.78 (d, 2H, H2/H9) ppm.

### *fac-[IrCl$_3$(DMSO)(dppz)]·2H$_2$O (Ir4a)*

Preparation as for **Ir1a** with 56.4 mg (0.2 mmol) of dipyrido[3,2-*a*:2',3'-c]phenanzine. Yield: 94.5 mg (68 %). Anal. Calcd. for C$_{20}$H$_{16}$Cl$_3$IrN$_4$OS·2H$_2$O (M = 695.0): C 34.56, H 2.90, N 8.06: Found C 34.4, H 3.2, N 7.8 %. LSIMS: *m/z* (%) = 625(6) [M-HCl]$^+$, 587(13) [M-2HCl]$^+$. $^1$H NMR (400 MHz, CD$_2$Cl$_2$, 30°C in the dark): δ = 3.51 (s, 6H, DMSO-CH$_3$), 8.06 (dd, 2H, H12/H13), 8.19 (dd, 2H, H3/H8), 8.42 (dd, 2H, H11/H14), 9.72 (d, 2H, H4/H7), 9.95 (d, 2H, H2/H9) ppm. $^1$H NMR (400 MHz, CD$_3$OD, 30°C): δ = 3.46 (s, 6H, DMSO-CH$_3$), 8.15 (dd, 2H, H12/H13), 8.31 (dd, 2H, H3/H8), 8.50 (dd, 2H, H11/14), 9.31 (d, 2H, H4/H7) 10.02 (d, 2H, H2/H9) ppm.

### *fac-[IrCl$_3$(DMSO)(dppn)]·2CH$_3$OH (Ir5a)*

Preparation as for **Ir1a** with 66.4 mg (0.2 mmol) of benzo[i]dipyrido[3,2-*a*:2',3'-c]phenanzine. Yield: 108.0 mg (70 %). Anal. Calcd. for C$_{24}$H$_{18}$Cl$_3$IrN$_4$OS·2CH$_3$OH (M = 773.2): C 40.39, H 3.39, N 7.25: Found C 40.8, H 3.4, N 7.3 %. LSIMS: *m/z* (%) = 674(5) [M-HCl]$^+$, 595(3) [M-HCl-DMSO]$^+$. $^1$H NMR (400 MHz, CD$_2$Cl$_2$, 30°C in the dark): δ = 3.39 (s, 6H, DMSO-CH$_3$), 7.62 (dd, 2H, H13/H14), 7.80 (dd, 2H, H3/H8), 8.21 (dd, 2H, H12/H15), 8.98 (s, 2H, H11/H16), 9.22 (d, 2H, H4/H7), 9.67 (d, H2/H9) ppm. $^1$H NMR (400 MHz, [D$^6$]DMSO, 30°C): δ = 3.33 (s, 6H, DMSO-CH$_3$), 7.73 (d, 2H, H13/H14), 8.25 (dd, 2H, H3/H8), 8.36 (d, 2H, H12/15), 9.12 (s, 2H, H11/H16), 9.29 (d, 2H, H4/H7), 9.76 (d, 2H, H2/H9) ppm.

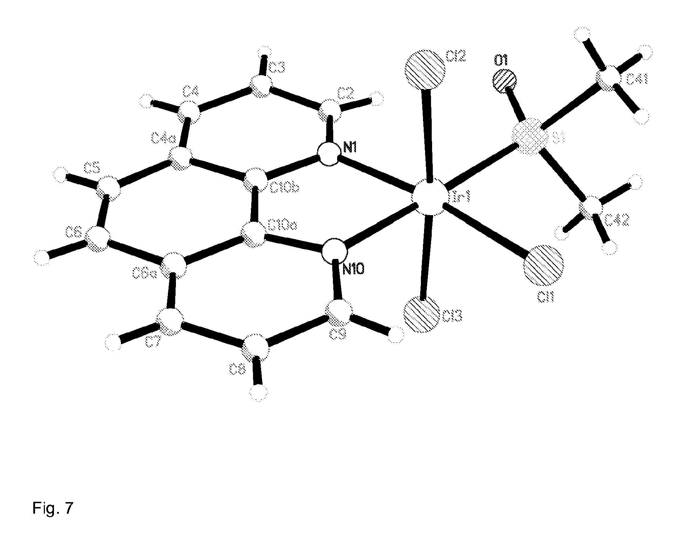

### *X-ray structural analysis of mer-[IrCl$_3$(DMSO)(phen)] Ir2b (Fig. 7)*

Crystals of **Ir2b** suitable for X-ray analyses were grown over a period of 7 days by slow evaporation of a solution of **Ir2a** in water/methanol. C$_{14}$H$_{14}$Cl$_3$IrN$_2$OS, M = 556.9, triclinic, space group P$\overline{1}$ (no. 2), α = 9.069(2), *b* = 11.989(2), *c* = 16.958(3) Å, α = 91.89(3), β = 103.92(3), γ = 92.68(3)$^0$, *V* = 1785.8(6) Å$^3$, *Z* = 4, *D*$_{calcd}$ = 2.071 g·cm$^{-3}$, μ(MoKα) = 8.043 mm$^{-1}$; Siemens P4 diffractometer using graphite-monochromated MoKα radiation (λ = 0.71073 Å). Intensity data

were collected using $\omega$ scans in the range $4° \leq 2\theta \leq 50°$ and the data were corrected semi-empirically for absorption using $\omega$ scan data. The structure was solved by direct methods with SHELX97 and refined against $F_0{}^2$ using SHELXL97 (G.M. Sheldrick, Göttingen, Germany, 1997). Anisotropic temperature factors were employed for non-hydrogen atoms and protons were included at geometrically calculated positions as riding atoms. The final R factors were $R_1 = 0.086$ for 2678 reflections with I > 2 $\sigma$(I) and $wR_2 = \{[\Sigma w(F_o{}^2 - F_o{}^2)^2 / \Sigma w(F_0)^2]\}^{1/2} = 0.240$, S (goodness-of-fit) = 0.915 for all 6087 independent reflections.

### Electronic spectra and DNA binding studies

UV/vis spectra were recorded with an Analytik Jena SPECORD 20 spectrometer and CD spectra with a Jasco J-715 instrument in the range 220 - 500 nm for 1:10 complex/[DNA] mixtures [complex = 20 $\mu$M, DNA concentration in M(Nucleotide) = 200 $\mu$M] in a 10 mM phosphate buffer at pH 7.2. To assure solubility of the complexes, 1 % DMSO was added to all of the aqueous buffer solutions. Thermal denaturation temperatures $T_m$ of the 1:10 complex/[DNA] mixtures were measured by recording melting curves at 1°C steps for the wavelength 260 nm on the SPECORD 200 spectrometer equipped with a Peltier temperature controller. $T_m$ values were calculated by determining the midpoints for melting curves from first-order derivatives and are estimated to be accurate within $\pm$ 1°C. Concentrations of CT DNA were determined spectrophotometrically using the molar extinction coefficient $\varepsilon_{260} = 6600$ M$^{-1}$ cm$^{-1}$.

### Mass spectrometry and NMR spectroscopy

LSIMS spectra (LSIMS = liquid secondary ion mass spectrometry) were registered for the mass range $m/z <$ 3000 with a Fisons VG Autospec employing a caesium ion gun (voltage 17 kV) and 3-nitrobenyzl alcohol as the liquid matrix. A Bruker DRX 400 was employed to record $^1$H and $^{13}$C NMR spectra with chemical shifts reported as $\delta$ values relative to the signal of the deuterated solvent.

The fac isomers of **Ir1a - Ir5a** are stable in light-protected $CD_2Cl_2$ solution but, with the exception of **Ir5a,** isomerize rapidly to a mixture of the fac and mer isomers in the presence of light. In contrast, solutions of the *fac* isomers in the polar solvents $D_2O$ and $CD_3OD$ are stable under such conditions. The isomer *mer*-[IrCl$_3$(DMSO-$\kappa S$)(phen)] **Ir2b** was, however, isolated by slow evaporation of an $H_2O/CH_3OH$ solution of **Ir2a** and characterized by X-ray structural analysis. UV/Vis and CD studies of the interaction of **Ir1a - Ir5a** with calf thymus DNA are in accordance with an absence of intercalation and $^1$H NMR studies indicate that the complexes do not react with the guanine N7 atom of 5'-GMP$^{2-}$.

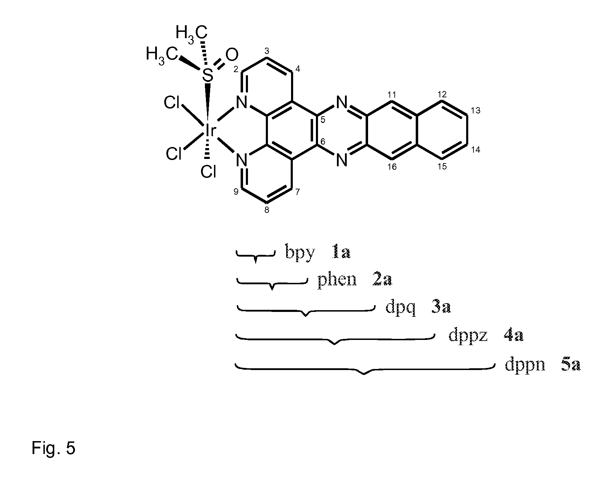

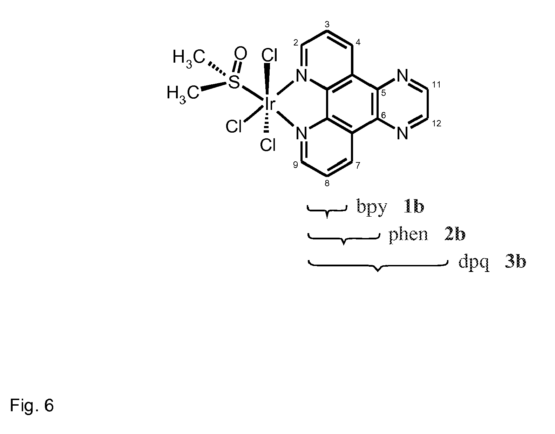

The structures of the fac complexes **Ir1a - Ir5a** and of the mer complexes **Ir1b - Ir3b** are shown in Figures 5 and 6.

### Example 4: Cytotoxic Measurements and cellular uptake studies fac-IrCl$_3$(DMSO)(pp) complexes and cellular uptake

The antiproliferative effects of the compounds **Ir1a - Ir5a** were determined as described in Example 2.

For cellular uptake studies, HT-29 and MCF-7 cells were grown until at least 70 % confluency in 175 cm$^2$ cell culture flasks [16]. Stock solutions of complexes **Ir1a - Ir5a** in DMSO were freshly prepared and diluted with cell culture medium to the desired concentrations (final DMSO concentrations: 0.1 % v/v, final complex concentration: 10 or 100 $\mu$M). The cell culture medium of the cell culture flasks was replaced with 10 mL of the cell culture medium solutions containing **Ir1a - Ir5a** (10 $\mu$M or 100 $\mu$M) and the flasks were incubated for 6 h at 37°C/5 % $CO_2$. The cell pellets were isolated, resuspended in 1 - 5 mL twice suspended water, lysed by using a sonotrode and approximately diluted using twice distilled water. The iridium content of the samples was determined by graphite furnace atomic absorption spectroscopy (GF-AAS) and the protein content of separate aliquots by the Bradford method. To correct for matrix effects in GF-AAS measurements, samples and standards were adjusted to the same protein concentration by dilution with twice distilled water. Prior to GF-AAS analysis 20 $\mu$L triton X-100 (1 %) and 20 $\mu$L hydrochloric acid were added to each 200 $\mu$L sample of the cell suspensions. Cellular uptake was expressed as ng iridium per mg cell protein for data obtained from 3 independent experiments. Conversion of the ng iridium/mg protein value to the micromolar cellular concentration was performed as described previously [16].

### Atomic Absorption Spectroscopy

A Vario 6 graphite furnace atomic absorption spectrometer (Analytik Jena) was employed for the Ir quantification using a wavelength of 208.9 nm with a bandpass of 0.5 nm. A deuterium lamp was used for background correction. Matrix containing standards were obtained by addition of an iridium stock solution (1 mg/mL Ir in 5 % HCl). 18 % hydrochloric acid solution was employed as a modifier [16]. Probes were injected at a volume of 25 $\mu$L into graphite tubes. Drying, pyrolysis and atomization in the graphite furnace was performed according to the conditions listed in Table 1. The detection limit for the method was 3.8 $\mu$g Ir L$^{-1}$. The mean AUC (area under curve) absorptions of duplicate injections were used throughout the study.

The IC$_{50}$ values and cellular uptake results are summarized in Tab. 2.

**Table 2** IC$_{50}$ values ($\mu$M) and cellular uptake (ng Ir/mg protein) of the complexes *fac*-[IrCl$_3$(DMSO)(pp)] **Ir1a - Ir5a** in comparison to other relevant compounds

| | | MCF-7 | | MCF-7 uptake | | HT-29 uptake | |
| --- | --- | --- | --- | --- | --- | --- | --- |
| Cpd. | pp | IC$_{50}$ | HT-29 IC$_{50}$ | 10 $\mu$M | 100 $\mu$M | 10 $\mu$M | 100 $\mu$M |
| **Ir1a** | bpy | > 100 | > 100 | n.d. | n.d. | 0.0 | 0.0 |
| **Ir2a** | phen | 4.6(0.5) | 4.6(0.2) | 0.0 | 74.4(13.5) | 0.0 | 21.2(5.2) |
| **Ir3a** | dpq | 5.5(0.9) | 6.1(0.7) | 0.3(0.3) | 640.5 (148.9) | 0.0 | 167.8(3.4) |
| **Ir4a** | dppz | 0.8(0.3) | 1.5(0.2) | 3.3(3.3) | 644.0 (149.1) | 11.9(3.0) | 1664.4 (473.1) |
| **Ir5a** | dppn | 0.21(0.11) | 1.3(0.4) | 19.3(0.8) | 180.8(2.7) | 37.4(8.9) | 226.1(26.6) |
| **6** | dppz | 2.3(0.4) | 7.4(0.9)[15] | n.d. | n.d. | 70.4(1.0) | n.d |
| **7** | dppn | 0.17(0.02) | 0.41(0.16) [15] | n.d. | n.d. | 149.6(7.8) | n.d |
| **dppz** | | 0.8(0.6) | 1.8(0.2) | n.d. | n.d. | n.d. | n.d. |
| **cisplatin** | | 2.0(0.3) | 7.0(2.0) | n.d. | n.d. | n.d. | n.d. |

n.d.: not determined 0.0: value below the detection limit

**6** = [($\eta^5$-C$_5$Me$_5$)IrCl(dppz)](CF$_3$SO$_3$) [5]

**7** = [($\eta^5$-C$_5$Me$_5$)Ir{(Me$_2$N)$_2$CS}(dppn)](CF$_3$SO$_3$) [5]

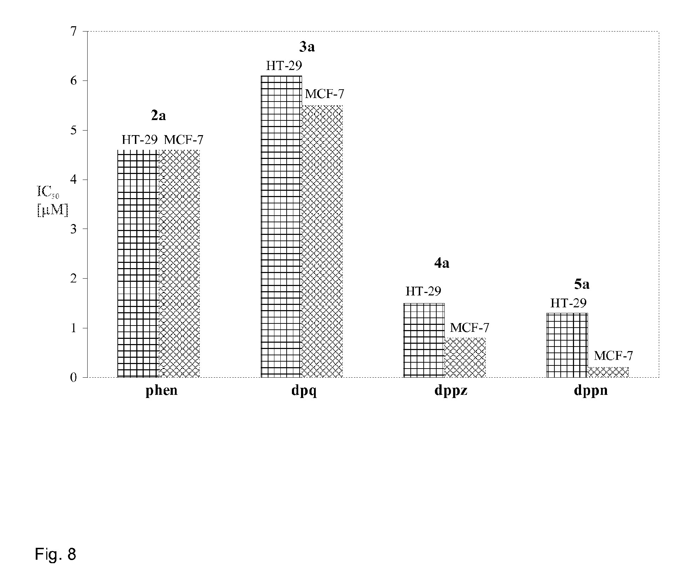

The cytotoxicity results are also depicted in Fig. 8 and indicate that, with the exception of the bpy complex **Ir1a,** the compounds of the typ fac-IrCl$_3$(DMSO)(pp) are potent cytotoxic agents, in particular the dppz and dppn complexes **Ir4a** and **Ir5a.**

In the following, the invention will be explained in more detail with reference to Figures 1 -8.

Figure Captions

Figure 1:    Structures of the trichloridorhodium(III) polypyridyl complexes *mer*-[RhCl$_3$(DMSO-$\kappa S$)(pp)] **Rh1a - Rh5a** (pp = bpy, phen, dpq, dppz, dppn)

Figure 2:    Structures of the fac isomers *fac*-[RhCl$_3$(DMSO-$\kappa S$)(pp)] **Rh1b** (pp = bpy) and **Rh2b** (pp = phen)

Figure 3:    Molecular structure of *fac*-[RhCl$_3$(DMSO-$\kappa S$)(bpy)] **Rh1b**

Figure 4:    IC$_{50}$ values for complexes **Rh1a - Rh5a** against the human cell lines MCF-7 (breast cancer) and HT-29 (colon cancer)

Figure 5:    Structures of the *fac* complexes **Ir1a - Ir5a**

Figure 6:    Structures of the *mer* complexes **Ir1b - Ir3b**

Figure 7:    Molecular structure of *mer*-[IrCl$_3$(DMSO)(pp)] **Ir2b** as established by X-ray structural analysis

Figure 8:    IC$_{50}$ values for the complexes **Ir2a - Ir5a** against the human cell lines MCF-7 (breast cancer) and HT-29 (colon cancer)

**References:**

[1] Mestroni, G. et al., Inorg. Chim. Acta 1998, 273, 62-71

[2] Colamarino, P. et al., J. Chem. Soc., Dalton Trans. 1976, 845-848

[3] Pruchnik, F. P., et al., Inorg. Chim. Acta 2002, 334, 59-66

[4] Medvetz, D. et al., J. Med. Chem. 2007, 50, 1703-1706

[5] Schäfer, S. et al., J. Organomet. Chem. 2007, 692, 1300-1309

[6] Schäfer, S. et al., Eur. J. Inorg. Chem. 2007,. 3034-3046

[7] Mura, P. et al., Inorg. Chim. Acta 2001, 312, 1-7
[8] Messori, L. et al., J. Inorg. Biochem. 2003, 95, 37-46
[9] Collins, J. G. et al., Inorg. Chem. 1998, 37, 3133-3141
[10] Delgadillo, A. et al., Helv. Chim. Acta 2003, 86, 2110-2120
[11] Yam, Y. W.-W. et al., J. Chem. Soc., Chem. Commun. 1995, 1191-1193
[12] James, B. R. et al., Can. J. Chem. 1980, 58, 399-408
[13] Sokol, V. et al., Sov. J. Coord. Chem. 1975, 1, 577-583
[14] Alessio, E. et al., Inorg. Chem. 1993, 32, 5756-5761
[15] Ott, I. et al., J. Med. Chem., 2005, 48, 622-629
[16] Ott, I. et al., ChemMedChem., 2007, 702-707

**Claims**

1. Octahedral metal (III) polypyridyl complexes of general formula (I)

$$Me(hal)_3(sol)(pp) \qquad (I)$$

where

Me represents rhodium or iridium
hal is a halogenide selected from the group consisting of chlorine, bromine, fluorine and iodine or a pseudohalogenide selected from the group consisting of SCN, NCO or $N_3$,
sol is a solvent selected from the group consisting of DMSO, $H_2O$, $CH_3OH$, DMF and $CH_3CN$
and
pp is a polypyridyl ligand selected from the group constisting of 2,2'-bipyridine (bpy), 1,10-phenanthroline (phen), dipyrido[3,2-f:2',3'-h]quinoxaline (dpq), dipyrido[3,2-a:2',3'-c]phenazine (dppz) and benzo[i]dipyrido[3,2-a:2',3'-c]phenazine (dppn), optionally substituted with one or more of the substituents selected from the group cons-tisting of hydroxy, -COOR, -SO$_3$H, -CHO, -CH$_3$, -CF$_3$, -OCH$_3$, -OC$_2$H$_5$, -NO$_2$, -CN, -NH$_2$, phenyl and halogenide, wherein R = H, -CH$_3$ or -C$_2$H$_5$,

and their physiologically tolerated isomers,
and hydrates, solvates and salts thereof.

2. Complexes of claim 1, wherein sol represents DMSO or $H_2O$.

3. Complexes of claim 1 or 2, wherein hal represents chlorine or bromine.

4. Complexes of anyone of claims 1 to 3, wherein the polypyridyl ligand represents phen, dpq, dppz or dppn, preferably dpq, dppz or dppn.

5. Complexes of anyone of claims 1 to 4 being hydrates or solvates.

6. Complexes of anyone of claims 1 to 5 being [Me(hal)$_3$(sol)(pp)](CH$_3$OH)$_m$ (H$_2$O)$_n$, wherein m is 0; 1 or 2 and n is 0; 1; 1,5; 2 or 3.

7. Complexes of anyone of claims 1 to 6 being the fac- and mer-isomers.

8. Complexes of anyone of claims 1 to 7, being

mer-RhCl$_3$(DMSO)(pp), fac-RhCl$_3$(DMSO)(pp),
mer-RhCl$_3$(DMSO-H$_2$O)(pp), fac-RhCl$_3$(DMSO-H$_2$O)(pp),
fac-RhCl$_3$(H$_2$O)(pp), mer-RhCl$_3$(H$_2$O)(pp),
fac-IrCl$_3$(DMSO)(pp), mer-IrCl$_3$(DMSO)(pp),
fac-IrCl$_3$(H$_2$O)(pp), mer-IrCl$_3$(H$_2$O)(pp),

fac-IrBr$_3$(H$_2$O)(pp), mer-IrBr$_3$(H$_2$O)(pp),
and their hydrates and solvates.

9. A pharmaceutical composition comprising as active substance one or more octahedral metal (III) polypyridyl complexes of claims 1 to 8.

10. Octahedral metal (III) polypyridyl complexes of claims 1 to 8 for prevention and treatment of cancer and its metastases, preferably breast cancer, colorectal cancer and neoplasia.

11. Use of one or more octahedral metal (III) polypyridyl complexes of claims 1 to 8 for the manufacture of a pharmaceutical composition for prevention and treatment of cancer and its metastases, preferably breast cancer, colorectal cancer and neoplasia.

12. A method for treating cancer which comprises administering to a patient suffering from cancer a therapeutically effective amount of one or more octahedral metal (III) polypyridyl complexes of claims 1 to 8.

Fig. 1

Fig. 2

Fig. 3

Fig. 4

Fig. 5

Fig. 6

Fig. 7

Fig. 8

**European Patent**
**Office**

## PARTIAL EUROPEAN SEARCH REPORT

which under Rule 63 of the European Patent Convention EP 07 15 0276
shall be considered, for the purposes of subsequent
proceedings, as the European search report

Application Number

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | PRUCHNIK, FLORIAN P. ET AL: "Rhodium(III) complexes with polypyridyls and pyrazole and their antitumor activity" INORGANICA CHIMICA ACTA , 334, 59-66 CODEN: ICHAA3; ISSN: 0020-1693, 2002, XP002477333 * the whole document * | 1-11 | INV. C07F15/00 A61K31/28 A61P35/00 |
| X | KULASINGAM, G. C.: "Complexes of 2,9-dimethyl-1,10-phenanthroline with rhodium(III) and iridium(III) halides" INORGANICA CHIMICA ACTA , 5(2), 180-2 CODEN: ICHAA3; ISSN: 0020-1693, 1971, XP002477334 * page 180 * | 1-8 | |
| X | MCKENZIE, ELWYN D. ET AL: "Rhodium(III) compounds with 1,10-phenanthroline and 2,2'-bipyridine" JOURNAL OF INORGANIC AND NUCLEAR CHEMISTRY , 32(1), 199-212 CODEN: JINCAO; ISSN: 0022-1902, 1970, XP002477335 * see page 205 * | 1-8 | |

-/--

TECHNICAL FIELDS
SEARCHED       (IPC)

C07F
A61K
A61P

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 21 April 2008 | Rinkel, Bert |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04E07)

## PARTIAL EUROPEAN SEARCH REPORT

European Patent Office

Application Number

EP 07 15 0276

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| X | BROOMHEAD, JOHN A. ET AL: "Iridium(III) and rhodium(III) mono-and bis(1,10-phenanthroline) complexes and the optical isomers of iridium(III), rhodium(III), chromium(III), and cobalt(III) dihalobis(1-10-phenanthroline) complex cations" INORGANIC CHEMISTRY , 10(9), 2002-9 CODEN: INOCAJ; ISSN: 0020-1669, 1971, XP002477336 * see Fig. 1 * | 1-8 | |
| A | WO 2004/012667 A (PURDUE RESEARCH FOUNDATION [US]; MORRISON HARRY [US]; MENON ELTON [US]) 12 February 2004 (2004-02-12) * claims * | 1-11 | TECHNICAL FIELDS SEARCHED (IPC) |

EPO FORM 1503 03.82 (P04C10)

**European Patent Office**

**INCOMPLETE SEARCH
SHEET C**

Application Number

EP 07 15 0276

Claim(s) searched completely:
        1-11

Claim(s) not searched:
        12

Reason for the limitation of the search (non-patentable invention(s)):

Article 53 (c) EPC - Method for treatment of the human or animal body by therapy

## ANNEX TO THE EUROPEAN SEARCH REPORT
## ON EUROPEAN PATENT APPLICATION NO.

EP 07 15 0276

This annex lists the patent family members relating to the patent documents cited in the above-mentioned European search report.
The members are as contained in the European Patent Office EDP file on
The European Patent Office is in no way liable for these particulars which are merely given for the purpose of information.

21-04-2008

| Patent document cited in search report | Publication date | Patent family member(s) | | Publication date |
|---|---|---|---|---|
| WO 2004012667 A | 12-02-2004 | AU | 2003257992 A1 | 23-02-2004 |
| | | CA | 2498231 A1 | 12-02-2004 |
| | | EP | 1546168 A2 | 29-06-2005 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Mestroni, G. et al.** *Inorg. Chim. Acta,* 1998, vol. 273, 62-71
- **Colamarino, P. et al.** *J. Chem. Soc., Dalton Trans.,* 1976, 845-848
- **Pruchnik, F. P. et al.** *Inorg. Chim. Acta,* 2002, vol. 334, 59-66
- **Medvetz, D. et al.** *J. Med. Chem,* 2007, vol. 50, 1703-1706
- **Schäfer, S. et al.** *J. Organomet. Chem,* 2007, vol. 692, 1300-1309
- **Schäfer, S. et al.** *Eur. J. Inorg. Chem,* 2007, 3034-3046
- **Mura, P. et al.** *Inorg. Chim. Acta,* 2001, vol. 312, 1-7
- **Messori, L et al.** *J. Inorg. Biochem.,* 2003, vol. 95, 37-46
- **Collins, J. G. et al.** *Inorg. Chem.,* 1998, vol. 37, 3133-3141
- **Delgadillo, A. et al.** *Helv. Chim. Acta,* 2003, vol. 86, 2110-2120
- **Yam, Y. W.-W. et al.** *J. Chem. Soc., Chem. Commun,* 1995, 1191-1193
- **James, B. R. et al.** *Can. J. Chem.,* 1980, vol. 58, 399-408
- **Sokol, V. et al.** *Sov. J. Coord. Chem.,* 1975, vol. 1, 577-583
- **Alessio, E. et al.** *Inorg. Chem.,* 1993, vol. 32, 5756-5761
- **Ott, I. et al.** *J. Med. Chem.,* 2005, vol. 48, 622-629
- **Ott, I. et al.** *ChemMedChem.,* 2007, 702-707